# EUROPEAN PATENT APPLICATION

(11) **EP 3 567 546 A1**
(43) Date of publication of application: **13.11.2019**
(21) Application number: 17889901.9
(22) Date of filing: 22.12.2017
(51) Int. Cl.: G06T 7/00, G01N 33/48

(54) **IMAGE ANALYSIS PROGRAM AND IMAGE ANALYSIS METHOD**

(30) Priority: 05.01.2017 JP 2017000298
(71) Applicant: Konica Minolta, Inc., Tokyo 100-7015 (JP)
(72) Inventor: MIMURA, Yusuke, Tokyo 100-7015 (JP)
(74) Representative: Henkel & Partner mbB
(86) International application number: PCT/JP2017/046063
(87) International publication number: WO 2018/128091

(57) **Abstract**

Provided are an image analysis program and an image analysis method which can reduce a burden on a diagnostician and properly extract a cell nucleus area corresponding to the diagnostician from an image for pathological diagnosis. An image analysis device for pathological diagnosis, which is provided with a display unit, an operation unit, and a storage unit, analyzes an image for pathological diagnosis by using a first parameter for determining a determination criterion of a cell nucleus for each diagnosis purpose and a second parameter for determining a determination criterion of a cell nucleus for each diagnostician. The image analysis device acquires an image which is for pathological diagnosis and is an image captured of a tissue sample, extracts candidate areas of a plurality of cell nuclei from the image for pathological diagnosis by using the first parameter, re-extracts one or a plurality of cell nuclei areas from the extracted candidate areas of the plurality of cell nuclei by using the second parameter, and allows the image for pathological diagnosis in which the re-extracted one or plurality of cell nucleus areas are discernable to be displayed on the display unit.

## Description

### Technological Field

The present invention relates to image analysis programs and image analysis methods, particularly to image analysis programs and image analysis methods, for extracting a diagnosis target corresponding to a diagnostician, from an image for pathological diagnosis.

### Background Art

Pathological diagnosis is one of medical diagnoses. In pathological diagnosis, a pathologist diagnoses a disease from a tissue piece sampled from a human body, and informs the necessity of medical treatment or surgery to a clinician. According to the condition of a patient and the result of the pathological diagnosis, a physician determines the policy of drug therapy for the patient, and a surgeon determines whether to perform surgery on the patient.

In the pathological diagnosis, a tissue specimen is prepared by cutting out a thin slice about several micrometers thick from a sample of tissue obtained by evisceration or needle biopsy, and a magnified image of the tissue specimen is observed under an optical microscope, which are commonly performed. To create such a tissue specimen, for example, a sample of tissue is dehydrated to be fixed and is made into a paraffin block, then a slice of a thickness suitable for a tissue section, is removed from the paraffin block, and paraffin wax is removed from the slice. In general, a tissue specimen is stained with one or more pigments before the observation, because light is hardly absorbed and scattered in a tissue specimen and a tissue specimen is nearly colorless and transparent.

Various staining methods have been proposed. Especially for tissue specimens, hematoxylin and eosin staining (HE staining), which is a staining method using two stains of hematoxylin and eosin, is used as a standard method. With hematoxylin staining, cell nuclei, calcified parts, cartilage tissues, bacteria and mucus are stained indigo blue or pale blue; and with eosin staining, cytoplasm, stromata, fibers, erythrocytes and keratinized cells are stained red or deep red. Pathologists make diagnoses on the basis of morphological information including a change of a cell nucleus in size or shape and a change of tissue in pattern, and of information about staining, in a microscopic image of a stained tissue specimen.

In addition, with the development of image digitalization technology, the following devices are coming into wide use also in the field of pathological diagnosis: the devices perform image processing on images for pathological diagnosis, which are digital color images input through a microscope or a digital camera, so as to detect or measure information (in particular, cell nuclei) necessary for pathological diagnosis to be made by pathologists, and then display the information.

An example of image processing methods for use in such devices for supporting pathological diagnosis, Patent Literature 1, which will be described below, discloses the following image processing method: the method includes a setup step of setting an initial contour of an object existing in an input image; an energy calculation step of calculating the energy of a contour point located on the initial contour set in the setup step and of calculating the energy of peripheral points given by moving the contour point to points located in a peripheral area; and a movement step of performing a minimum seeking on the energy calculated for the points in the energy calculation step and of moving the contour point to the minimum energy point. In this image processing method, processing of each of the energy calculation step and the movement step is repeated by using a processing result of the other step; and when calculating the energy in the energy calculation step, the energy calculation is performed by using a blurred input image at the initial stage and then energy calculations are performed by using input images which become gradually less blurred.

Further, Patent Literature 2, which will be described below, discloses the following medical-diagnosis support device: the medical-diagnosis support device includes: a training-data obtainment means for obtaining training data; an inference-means-candidate creation means for creating a plurality of candidates for an inference means, on the basis of the training data; an inference-performance evaluation means for evaluating the performance of each of the plurality of candidates for the inference means, on the basis of the training data; an information-validity evaluation means for evaluating the validity of information presented by each of the plurality of candidates for the inference means, on the basis of the training data; and an inference-means selection means for selecting an inference means from the plurality of candidates for the inference means, on the basis of the performance of each of the plurality of candidates for the inference means and the validity of information presented by each of the plurality of candidates for the inference means.

### Citation List

### Patent Literature

Patent Literature 1: Japanese Unexamined Patent Publication (JP-A) No. 2000-331143
Patent Literature 2: JP-A No. 2014-147659

### Summary of Invention

### Problem to be solved by the Invention

There are various methods for extracting an area indicating a cell nucleus from an image for pathological diagnosis. Machine learning is one of common methods for extracting a cell nucleus area from a pathological diagnosis image. To extract a cell nucleus area from a pathological diagnosis image by using machine learning, it is necessary that a machine learns cell nucleus areas in various kinds of pathological diagnosis image, as training examples. The machine learning using cell nucleus areas in pathological diagnosis images has the following problems.

The first problem is that a criterion for cell nucleus determination differs depending on diagnostic purpose. That is, even when a same pathological diagnosis image is used, cell nucleus areas to be extracted and cell nucleus areas to be ruled out from extraction targets differ depending on diagnostic purpose. Therefore, a use of cell nuclei determined with unknown diagnostic purpose, worsens the accuracy of machine learning.

The second problem is that a criterion for cell nucleus determination varies depending on a pathologist. That is, the condition of samples of tissue for pathological diagnosis can change due to a method of preparing the samples, a method of staining the samples and other factors, and further, a pathological diagnosis image can greatly change due to shooting conditions. Therefore, the criterion for determining cell nuclei in a pathological diagnosis image widely varies depending on a pathologist. Accordingly, even when pathological diagnosis images of the same tissue sample are used for machine learning, a result of extraction of cell nuclei changes in accordance with the condition of the tissue sample and the criterion for cell nucleus determination, which worsens the accuracy of the machine learning.

In view of the problems, in order to train a device for supporting pathological diagnosis, by machine learning valuable to respective users, it is necessary that the device is trained by machine learning according to the criterion for cell nucleus determination corresponding to each diagnosis purpose and the criterion for cell nucleus determination corresponding to each diagnostician like a pathologist, and adjusts a parameter to an appropriate value for each diagnosis purpose and for each diagnostician. To achieve that, it is necessary that the device is trained by machine learning using a huge amount of parameters, for a large amount of man-hours, which increases the burden on diagnosticians and increases the time necessary for the machine learning.

The present invention has been made in view of the above-described problems, one of main objectives of the present invention is to present an image analysis program and an image analysis method, capable of extracting appropriate cell nucleus areas corresponding to a diagnostician, from an image for pathological diagnosis, with reducing the burden on diagnosticians.

### Solution to Problem

One aspect of the present invention is an image analysis program to be executed in a device that includes a display unit, an operation unit and a storage unit, for analyzing images for pathological diagnosis by using a first parameter for determining a criterion for cell nucleus determination for each diagnosis purpose and a second parameter for determining a criterion for cell nucleus determination for each diagnostician, both recorded in the storage unit. The program comprises instructions which causes the device to carry out: a step of first analyzing a pathological diagnosis image, including acquiring a pathological diagnosis image prepared by shooting a tissue specimen, and extracting a plurality of possibles for a cell nucleus area from the pathological diagnosis image, by using the first parameter; a step of second analyzing a pathological diagnosis image, including further extracting one or more cell nucleus areas from the plurality of possibles for a cell nucleus area that were extracted, by using the second parameter; and a step of first carrying out display control, including causing the display unit to display the pathological diagnosis image with the one or more cell nucleus areas that were extracted made recognizable.

One aspect of the present invention is an image analysis method for use in a device that includes a display unit, an operation unit and a storage unit, for analyzing images for pathological diagnosis by using a first parameter for determining a criterion for cell nucleus determination for each diagnosis purpose and a second parameter for determining a criterion for cell nucleus determination for each diagnostician, both recorded in the storage unit. The image analysis method comprises: a step of first analyzing a pathological diagnosis image, including acquiring a pathological diagnosis image prepared by shooting a tissue specimen, and extracting a plurality of possibles for a cell nucleus area from the pathological diagnosis image, by using the first parameter; a step of second analyzing a pathological diagnosis image, including further extracting one or more cell nucleus areas from the plurality of possibles for a cell nucleus area that were extracted, by using the second parameter; and a step of first carrying out display control, including causing the display unit to display the pathological diagnosis image with the one or more cell nucleus areas that were extracted made recognizable.

### Advantageous Effects of Invention

Image analysis programs and image analysis methods according to the present invention allow appropriate extraction of cell nucleus areas corresponding to a diagnostician, from an image for pathological diagnosis, with reducing the burden on diagnosticians.

The reason is that the following control operations are performed in a device for analyzing an image for pathological diagnosis. That is, this device acquires a pathological diagnosis image prepared by shooting a tissue specimen and extracts a plurality of possibles for a cell nucleus area from the pathological diagnosis image, by using the first parameter. The device further extracts one or more cell nucleus areas from the plurality of possibles for a cell nucleus area that were extracted, by using the second parameter, and causes the display unit to display the pathological diagnosis image with the one or more cell nucleus areas that were extracted made recognizable.

### Brief Description of Drawings

FIG. 1 is a schematic diagram illustrating an outline of operations of an image analysis device for pathological diagnosis, according to an embodiment of the present invention;
FIG. 2 is a schematic diagram illustrating a constitution of an image processing system for pathological diagnosis, according to the first embodiment of the present invention;
FIG. 3A is a block diagram illustrating a constitution of the image analysis device for pathological diagnosis, according to the first embodiment of the present invention;
FIG. 3B is a block diagram illustrating a constitution of the image analysis device for pathological diagnosis, according to the first embodiment of the present invention;
FIG. 4 is a flowchart illustrating operations of the image analysis device for pathological diagnosis, according to the first embodiment of the present invention;
FIG. 5 is a diagram illustrating an example of a possible-area display screen (for dead cell determination) according to the first embodiment of the present invention;
FIG. 6 is a diagram illustrating an example of a possible-area display screen (size-based setting) according to the first embodiment of the present invention;
FIG. 7 is a diagram illustrating an example of a possible-area display screen (density-based setting) according to the first embodiment of the present invention;
FIG. 8 is a diagram illustrating an example of a possible-area display screen (edge-strength-based setting) according to the first embodiment of the present invention;
FIG. 9 is a diagram illustrating an example of a possible-area display screen (feature-based setting) according to the first embodiment of the present invention;
FIG. 10 is a flowchart illustrating operations of the image analysis device for pathological diagnosis, according to the second embodiment of the present invention;
FIG. 11 is a diagram illustrating an example of a sample-image display screen (size-based setting) according to the second embodiment of the present invention;
FIG. 12 is a diagram illustrating an example of a sample-image display screen (density-based setting) according to the second embodiment of the present invention;
FIG. 13 is a diagram illustrating an example of a sample-image display screen (edge-strength-based setting) according to the second embodiment of the present invention;
FIG. 14 is a flowchart illustrating steps of conventional image analysis for pathological diagnosis;
FIG. 15 is a table of factors in coloration change of images, in a case of extraction of a cell nucleus from a pathological diagnosis image;
FIG. 16A is a diagram for illustrating an example of images which are different in coloration, in a case of extraction of a cell nucleus from a pathological diagnosis image;
FIG. 16B is a diagram for illustrating an example of images which are different in coloration, in a case of extraction of a cell nucleus from a pathological diagnosis image;
FIG. 17 is a diagram illustrating the difference of the criteria for cell nucleus determination in accordance with diagnostic purpose, in a case of extraction of a cell nucleus from a pathological diagnosis image;
FIG. 18 is a diagram illustrating the difference of the criteria for cell nucleus determination (in the case of dead cells) in accordance with diagnostic purpose, to be caused when a cell nucleus is extracted from a pathological diagnosis image;
FIG. 19 is a diagram illustrating the variation in the criteria for cell nucleus determination in accordance with a pathologist, to be caused when a cell nucleus is extracted from a pathological diagnosis image;
FIG. 20A is a diagram for illustrating the variation in the criteria for cell nucleus determination (in the case of separating superimposed cell nuclei) in accordance with a pathologist, to be caused when a cell nucleus is extracted from a pathological diagnosis image; and
FIG. 20B is a diagram for illustrating the variation in the criteria for cell nucleus determination (in the case of separating superimposed cell nuclei) in accordance with a pathologist, to be caused when a cell nucleus is extracted from a pathological diagnosis image.

### Description of Embodiments

As described in Background Art, when a device for supporting pathological diagnosis extracts cell nuclei from a pathological diagnosis image by using machine learning, it is necessary that the device addresses the difference of the criteria for cell nucleus determination according to diagnostic purpose and the variation in the criteria for cell nucleus determination according to a pathologist. However, because of preparation of parameters for machine learning in accordance with various kinds of diagnostic purpose, the number of the parameters swells, and moreover, because of machine learning in consideration of the variation in the criteria for cell nucleus determination according to a pathologist, the burden on pathologists and the time necessary for additional learning increase, resulting in serious problems. A description is given of the problems with reference to drawings.

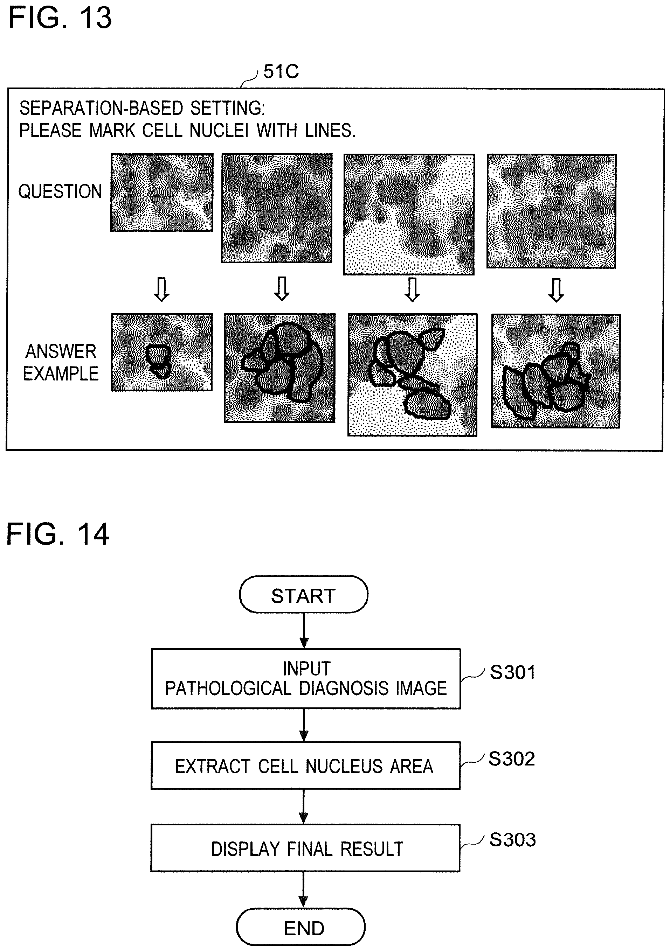

FIG. 14 is a flowchart illustrating steps of conventional image analysis for pathological diagnosis. In the conventional analysis, in response to input of an image for pathological diagnosis into a device for supporting pathological diagnosis (S301), the device analyzes the pathological diagnosis image to extract cell nucleus areas (S302), and displays the extracted cell nucleus areas as the final result (S303). In other words, in the conventional image analysis for pathological diagnosis, the device for supporting pathological diagnosis extracts cell nucleus areas from the pathological diagnosis image by using machine learning parameters prepared in advance, and displays them as the final result. With this method, the given extraction result does not reflect the difference of the criteria for cell nucleus determination according to diagnostic purpose and the variation in the criteria for cell nucleus determination according to a pathologist.

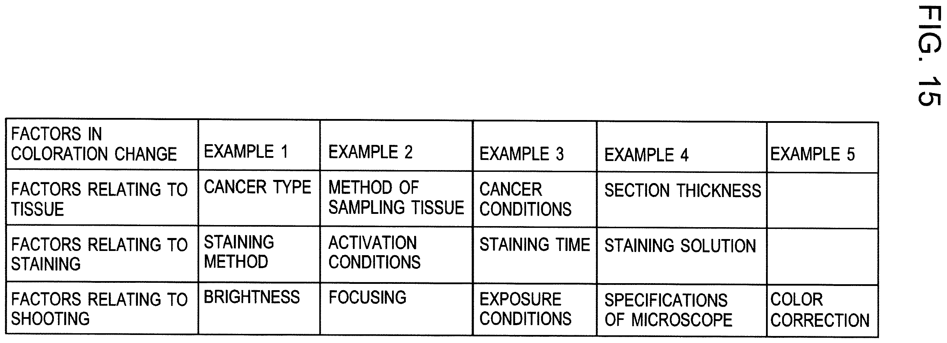

FIG. 15 is a table of factors in coloration change of pathological diagnosis images. The factors in coloration change of pathological diagnosis images include factors relating to tissue, factors relating to staining and factors relating to shooting. The factors relating to tissue include the type of cancer, the method of sampling tissue, the conditions of cancer and the thickness of a section. The factors relating to staining includes the staining method, the activation conditions, the staining time and the staining solution. The factors relating to shooting includes the brightness, the focusing, the exposure conditions, the specifications of a microscope and the color correction.

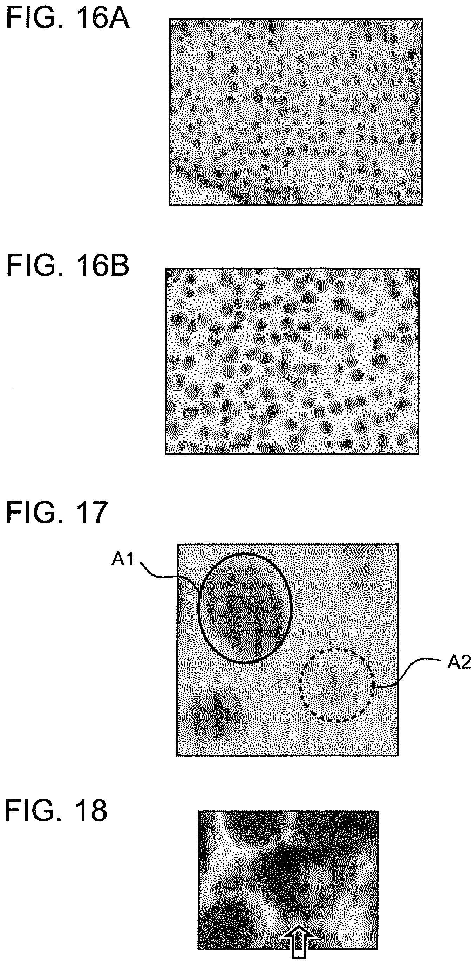

FIGs. 16A and 16B illustrate an example of pathological diagnosis images, which are different in coloration. FIGs. 16A and 16B are both pathological diagnosis images of the same breast cancer, but the images are quite different in coloration from each other (colors in the figures are expressed by shades of grays, since these figures are shown in a black-and-white or binary manner).

FIG. 17 is an example of a pathological diagnosis image for illustrating the difference of the criteria for cell nucleus determination in accordance with diagnostic purpose. The part A1 enclosed with the solid line in the figure is a part that a pathologist determines as a cell nucleus generally. On the other hand, the part A2 enclosed with the broken line in the figure is a part indicating a cell membrane of a cell. There is a need to extract the part A2 in a case of a diagnosis that is necessary to observe even cell membranes, but there is no need to extract the part A2 in a case of a diagnosis that is not necessary to observe cell membranes.

FIG. 18 is another example of a pathological diagnosis image for illustrating the difference of the criteria for cell nucleus determination in accordance with diagnostic purpose, and the image was shot with attention to dead cells. The part pointed by the arrow in the figure indicates a cell nucleus and the cell nucleus has begun to rupture. Therefore, it can be considered that a cell including the cell nucleus is dead. Similarly to the above, there is a need to extract the part in a case of a diagnosis that is necessary to observe even dead cells, but there is no need to extract the part in a case of a diagnosis that is not necessary to observe dead cells.

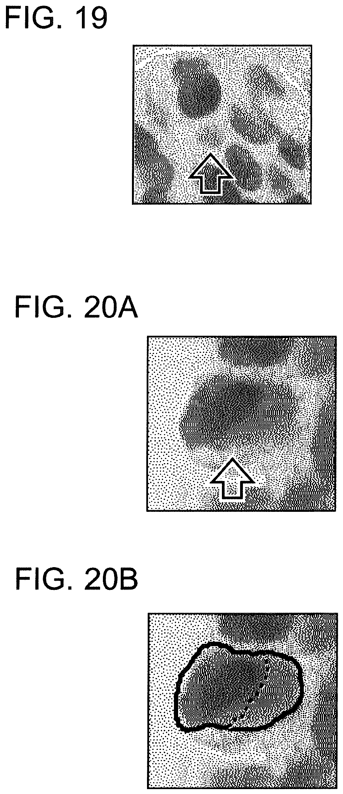

FIG. 19 is a diagram of an example of a pathological diagnosis image for illustrating the variation in the criteria for cell nucleus determination in accordance with a pathologist. As for the part pointed by the arrow in the figure (the part in which the color changes weakly), the determination whether to extract the part as a cell nucleus differs depending on a pathologist.

FIGs. 20A and 20B are diagrams of another example of a pathological diagnosis image for illustrating the variation in the criteria for cell nucleus determination in accordance with a pathologist, wherein FIG. 20A is the original image and FIG. 20B is a diagram illustrating an example that a line separating superimposed cell nuclei from each other was added to the original image. As for the nucleus or nuclei at the center of the original image of FIG. 20A (the part pointed by the arrow), one pathologist may determine the part enclosed with the solid line in FIG. 20B as one cell nucleus, and another pathologist may separate the part enclosed with the solid line, with the broken line, and determine the separated pieces as two cell nuclei.

As described above, a cell nucleus area or areas to be extracted from a pathological diagnosis image, differ in accordance with diagnostic purpose, and even for the same diagnostic purpose, a cell nucleus area or areas to be extracted from a pathological diagnosis image, differ in accordance with a pathologist. Further, there are many factors in coloration change of a pathological diagnosis image as described above, the appearance of cells change together with the coloration change of a pathological diagnosis image. Therefore, in a conventional device for supporting pathological diagnosis, the variation in the extraction result in accordance with diagnosis purpose or a pathologist becomes greater, which results in that the device becomes to fail to extract a cell nucleus area to be extracted from a pathological diagnosis image or becomes to extract a cell nucleus area not to be extracted.

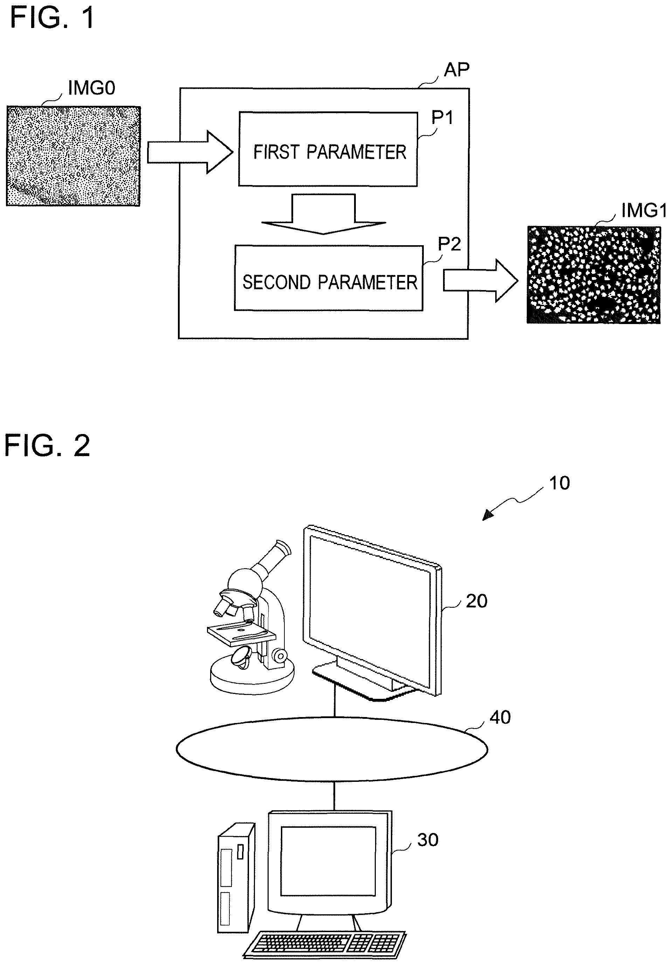

In view of that, an embodiment of the present invention provides a method to extract a cell nucleus area from a pathological diagnosis image by using machine learning, wherein a parameter for determining a criterion for cell nucleus determination for each diagnostic purpose has been prepared by machine learning in advance. If additional machine learning is made so as to adjust the parameter according to the criterion for cell nucleus determination for each diagnostician like a pathologist, it may increase the burden on diagnosticians and the time necessary for the learning. In view of that, as illustrated in FIG. 1, additionally to machine learning parameters for extracting cell nucleus areas from a pathological diagnosis image in accordance with diagnosis purpose (first parameters P1 for determining the criterion for cell nucleus determination for each diagnosis purpose), there is prepared in advance a parameter that can be adjusted according to a change of the determination criterion in accordance with a diagnostician (second parameter P2 for determining a criterion for cell nucleus determination for each diagnostician). Hereinafter, the first parameter P1 and the second parameter P2 are referred to as a basic parameter and a specific parameter, respectively. After that, as illustrated in FIG. 1, image analysis device for pathological diagnosis AP acquires pathological diagnosis image IMG0 given by shooting a tissue specimen, and uses an extraction result given by extracting multiple possibles for a cell nucleus area from pathological diagnosis image IMG0 by using the basic parameter, to extract one or more cell nucleus areas from the possibles by using the specific parameter. In other words, the second extraction of one or more cell nucleus areas so as to handle the variation of the criterion for cell nucleus determination in accordance with a pathologist is made only from the result of extraction given by extracting possibles for a cell nucleus area from the pathological diagnosis image by using the basic parameter. After that, the device displays pathological diagnosis image IMG1 with the one or more extracted cell nucleus areas made recognizable. With this method, one or more cell nucleus areas corresponding to a diagnostician can be extracted properly from a pathological diagnosis image, with reducing the burden on diagnosticians.

In concrete terms, an image analysis device for pathological diagnosis, equipped with a display unit, an operation unit and a storage unit, for analyzing images for pathological diagnosis by using a basic parameter and a specific parameter recorded in the storage unit, carries out the following operations. The image analysis device for pathological diagnosis carries out a process of first analyzing a pathological diagnosis image, including acquiring a pathological diagnosis image prepared by shooting a tissue specimen and extracting a plurality of possibles for a cell nucleus area from the pathological diagnosis image, by using the basic parameter. The device further carries out a process of second analyzing a pathological diagnosis image, including further extracting one or more cell nucleus areas from the plurality of extracted possibles for a cell nucleus area, by using the specific parameter; and a process of first carrying out display control, including causing the display unit to display the pathological diagnosis image with the one or more extracted cell nucleus areas made recognizable.

In the above processes, the image analysis device for pathological diagnosis may correct the specific parameter to a value corresponding to a diagnostician on the basis of a result of operations performed by the diagnostician on possibles for a cell nucleus area extracted from the pathological diagnosis image by using the basic parameter or one or more samples of a cell nucleus image prepared in advance. With this process, the additional machine learning so as to adjust the parameter according to the criterion for cell nucleus determination for each diagnostician can be made by using a limited number of training samples, which reduces the burden of diagnosticians and further reduces the amount of man-hours necessary for the machine learning. For example, after the process of first analyzing a pathological diagnosis image, the image analysis device for pathological diagnosis may carry out a process of second carrying out display control, including causing the display unit to display one or more of the plurality of extracted possibles for a cell nucleus area, so that a selection from the one or more of the plurality of possibles or addition of information onto the one or more of the plurality of possibles can be made; a process of carrying out operations control, including receiving an operation to make a selection from the one or more of the plurality of possibles or an operation to make an addition of information onto the one or more of the plurality of possibles, by using the operation unit; and a process of making a parameter correction, including using a result of the operation to make a selection from the one or more of the plurality of possibles or a result of the operation to make an addition of information onto the one or more of the plurality of possibles, to correct the specific parameter, and recording the specific parameter into the storage unit. In the process of second analyzing a pathological diagnosis image, one or more cell nucleus areas may be extracted from the plurality of extracted possibles for a cell nucleus area, by using the corrected specific parameter. Alternatively, before the process of first analyzing a pathological diagnosis image, the image analysis device for pathological diagnosis may carry out a process of second carrying out display control, including causing the display unit to display one or more samples of a cell nucleus image, so that a selection from the one or more samples of a cell nucleus image or addition of information onto the one or more samples of a cell nucleus image can be made; a process of carrying out operations control, including receiving an operation to make a selection from the one or more samples of a cell nucleus image or an operation to make an addition of information onto the one or more samples of a cell nucleus image, by using the operation unit; and a process of making a parameter correction, including using a result of the operation to make a selection from the one or more samples of a cell nucleus image or a result of the operation to make an addition of information onto the one or more samples of a cell nucleus image, to correct the specific parameter, and recording the specific parameter into the storage unit. In the process of second analyzing a pathological diagnosis image, one or more cell nucleus areas may be extracted from the plurality of extracted possibles for a cell nucleus area, by using the corrected specific parameter.

### First Embodiment

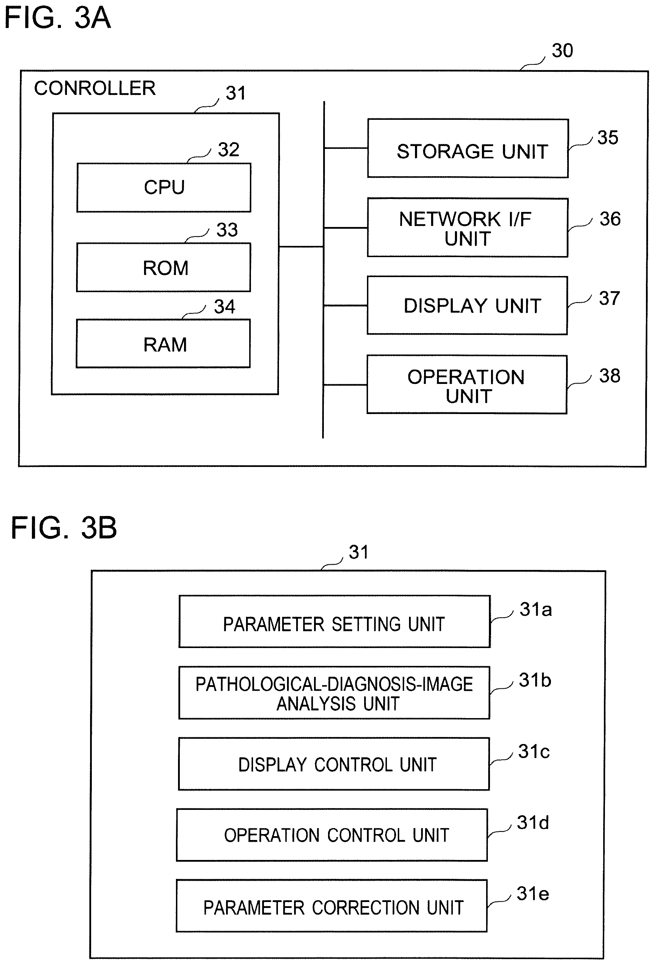

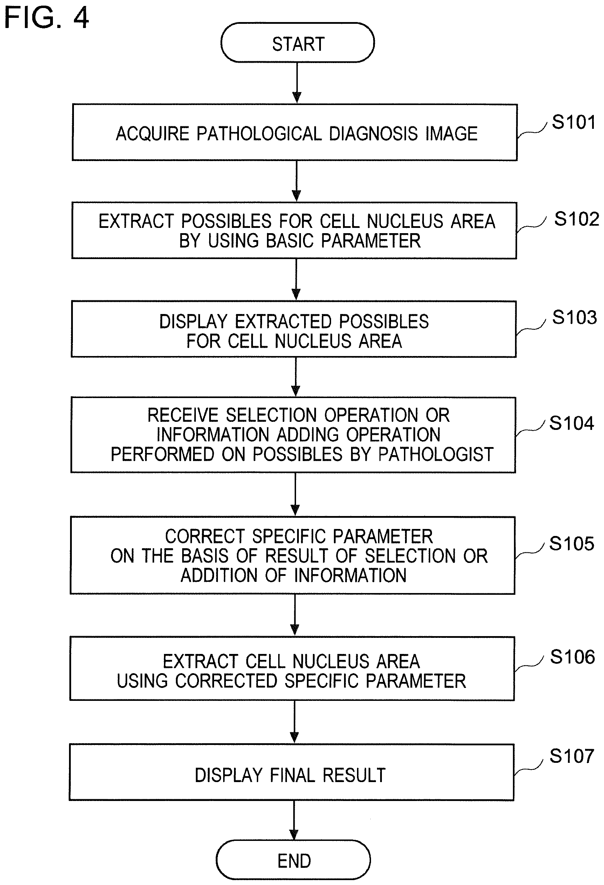

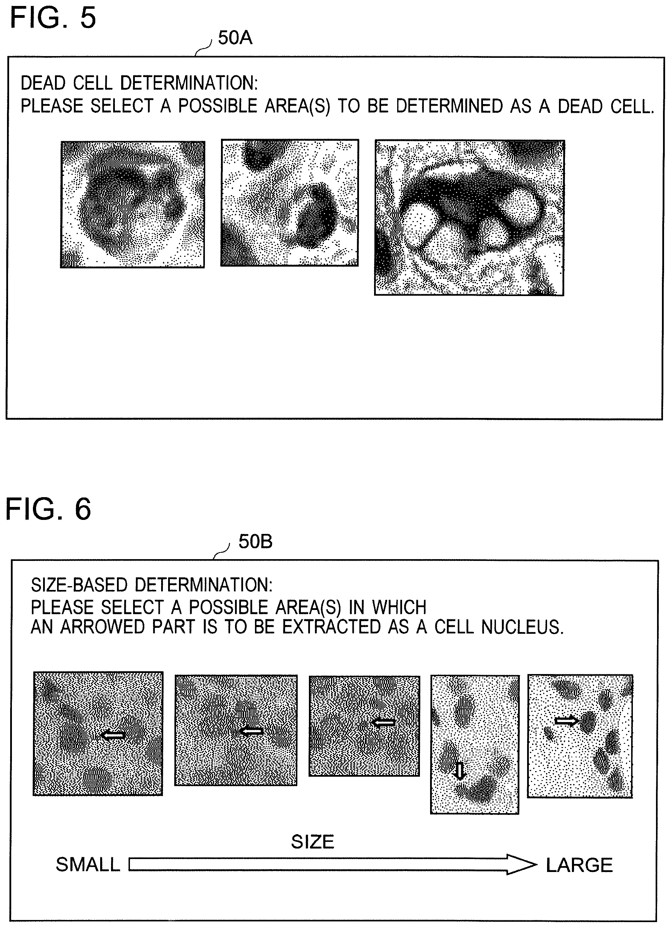

In order to describe embodiments of the present invention in more in detail, a description is given of an image analysis program and an image analysis method according to a first embodiment of the present invention, with reference to FIG. 2 through FIG. 9. FIG. 2 is a schematic diagram illustrating a constitution of an image processing system for pathological diagnosis, according to the present embodiment. Each of FIG. 3A and FIG. 3B is a block diagram illustrating a constitution of an image analysis device for pathological diagnosis. FIG. 4 is a flowchart illustrating operations of the image analysis device for pathological diagnosis. Each of FIGs. 5 to 9 is a diagram illustrating an example of a possible-area display screen to be used for correcting the specific parameter.

As illustrated in FIG. 2, image processing system for pathological diagnosis 10 according to the present embodiment includes image acquisition device for pathological diagnosis 20 and image analysis device for pathological diagnosis 30. These are communicably connected to each other via communication network 40, where examples of the communication network 40 include a LAN (Local Area Network) defined by a standard, such as Ethernet (registered trademark), Token Ring or FDDI (Fiber-Distributed Data Interface). In a case that image data of a pathological diagnosis image acquired by image acquisition device for pathological diagnosis 20 is presented to image analysis device for pathological diagnosis 30 through a USB (Universal Serial Bus) memory, there is no need to connect image acquisition device for pathological diagnosis 20 and image analysis device for pathological diagnosis 30 through communication network 40.

Image acquisition device for pathological diagnosis 20 include: an optical microscope for shooting a tissue specimen created by cutting out a thin slice about several micrometers thick from a sample of tissue obtained by evisceration or needle biopsy; a monitor for displaying a pathological diagnosis image shot by the optical microscope; and a controller for controlling them and for creating and outputting image data of the pathological diagnosis image.

Image analysis device for pathological diagnosis 30 is a computing device like a personal computer, and includes controller 31, storage unit 35, network I/F unit 36, display unit 37 and operation unit 38, as illustrated in FIG. 3A.

Controller 31 includes CPU (Central Processing Unit) 32, and memories including ROM (Read Only Memory) 23 and RAM (Random Access Memory) 24, and these are connected through a bus. ROM 33 stores programs and other data. RAM 34 stores data necessary for control operations executed by CPU 32, data that need to be stored temporally in the control operations, and other data. CPU 32 controls the whole operations of image analysis device for pathological diagnosis 30 by loading a control program stored in ROM 33 or storage unit 35 onto RAM 34 and executing the control program.

As illustrated in FIG. 3B, controller 31 works as parameter setting unit 31a, pathological-diagnosis-image analysis unit 31b, display control unit 31c, operation control unit 31d, parameter correction unit 31e and the like.

Parameter setting unit 31a sets a parameter for extracting possibles for a cell nucleus area corresponding to diagnosis purpose from a pathological diagnosis image (the basic parameter for determining a criterion for cell nucleus determination for each diagnosis purpose) and a parameter for extracting one or more cell nucleus areas corresponding to a diagnostician like a pathologist (hereinafter, referred to as a pathologist) from the extracted possibles for a cell nucleus area (the specific parameter for determining a criterion for cell nucleus determination for each pathologist); and records them into storage unit 25 or another storage device. As the basic parameter, for example, one or more selected from the cell nucleus size, the staining density of a cell nucleus, the edge strength of the contour of a cell nucleus, a numerical value of the feature like the likelihood ratio, and others, may be used. As for the specific parameter, for example, one or more selected from the cell nucleus size, the staining density of a cell nucleus, the edge strength of the contour of a cell nucleus, a numerical value of the feature like the likelihood ratio, the pattern to be determined as a dead cell and others, may be used. As for the initial values of the parameters, standard values prepared in advance may be assigned to the parameters, or values obtained by selection or input of a pathologist or another operator though operation unit 38 may be assigned to the parameters. Further, the item or items used as the specific parameter may be included in the item or items used as the basic parameter, a part of the items used as the specific parameter may be common to an item or items used as the basic parameter, or the item or items used as the specific parameter may different form the item or items used for the basic parameter. In a case that the item or items used as the specific parameter is included in the item or items used as the basic parameter, or that a part of the items used as the specific parameter is common to an item or items used as the basic parameter, the initial value of each common item of the specific parameter may be the same as that of the basic parameter.

Pathological-diagnosis-image analysis unit 31b acquires a pathological diagnosis image given by shooting a tissue specimen, from image acquisition device for pathological diagnosis 20, carries out the edge enhancement on the obtained pathological diagnosis image by using a known technique like a relaxation method, a zero-crossing cell, a Canny cell, or a non-extremum suppression method, and detects the contours by using a known technique like a Hough transform or SNAKE method. Then, pathological-diagnosis-image analysis unit 31 performs image processing on the detected contours by using the above-described basic parameters, to extract multiple possibles for a cell nucleus area corresponding diagnostic purpose from the pathological diagnosis image. Then, pathological-diagnosis-image analysis unit 31 performs image processing on the extracted possibles for a cell nucleus area by using the specific parameters, to extract one or more cell nucleus areas corresponding a diagnostician.

Display control unit 31c, in response to extraction of the multiple possibles for a cell nucleus area with pathological-diagnosis-image analysis unit 31b, classifies the extracted possibles for a cell nucleus area into groups for adjusting the specific parameter to a value corresponding to a diagnostician like a pathologist. In this process, two or more groups may include the same possible for a cell nucleus area. Then, display control unit 31c causes display unit 37 to display the possibles for a cell nucleus area in each group so that a selection from the possibles can be made (in other words, so as to allow a diagnostician like a pathologist to select one or more of the possibles for a cell nucleus area in each group). Alternatively, from the extracted possibles for a cell nucleus area, display control unit 31c determines one or more possibles for a cell nucleus area to be used for adjusting the specific parameter to a value corresponding to a diagnostician like a pathologist, and causes display unit 37 to display the one or more determined possibles for a cell nucleus area so that an addition of information onto the one or more of the determined possibles for a cell nucleus area can be made (in other words, so as to allow a diagnostician like a pathologist to add information on the one or more determined possibles for a cell nucleus area with operation unit 38). Further, display control unit 31c, in response to extraction of one or more cell nucleus areas corresponding to a pathologist from possibles for a cell nucleus area with pathological-diagnosis-image analysis unit 31b, causes display unit 37 to display the pathological diagnosis image with the one or more extracted cell nucleus areas made recognizable, as a final result. To make the one or more extracted cell nucleus areas recognizable, display control unit 31c may, for example, cause display unit 37 to display the pathological diagnosis image with the one or more extracted cell nucleus areas enhanced. Examples of a method to enhance the extracted cell nucleus area, include: changing the brightness or color of the area to be emphasized in a pathological diagnosis image, blinking the area to be emphasized, and displaying a frame or marker indicating the area to be emphasized together with a pathological diagnosis image. Alternatively, to make the extracted one or more cell nucleus areas recognizable, display control unit 31c may cause display unit 37 to display the one or more extracted cell nucleus areas in place of causing display unit 37 to display the whole pathological diagnosis image. Display control unit 31c further cause display unit 37 to display a screen for causing an operator to perform setting or selection operation for the basic parameter or the specific parameter, as needed.

Operation control unit 31d, in response to selection operation performed on the possibles for a cell nucleus area displayed by display unit 37, by a pathologist through operation unit 38, receives the selection operation and informs about the result of the selection operation to parameter correction unit 31e. Further, operation control unit 31d, in response to information-adding operation performed on the possibles for a cell nucleus area displayed by display unit 37, by a pathologist through operation unit 38, receives the information-adding operation and informs about the result of the information-adding operation to parameter correction unit 31e. Operation control unit 31d may further receive setting operations and selection operations for the basic parameter or the specific parameter, as needed.

Parameter correction unit 31e receives a result of the selection operation or the information-adding operation performed on the one or more possibles for a cell nucleus area by a pathologist, from operation control unit 31d. Parameter correction unit 31e corrects the specific parameter to a value corresponding to a pathologist on the basis of the difference between the selected possible or possibles for a cell nucleus area and the unselected possible or possibles for a cell nucleus area, or corrects the specific parameter to a value corresponding to a pathologist on the basis of the contents of the added information. Parameter correction unit 31e then records the corrected specific parameter into storage unit 35 or another storage device. For example, when receiving a result of the selection operation performed on the possibles for a cell nucleus area by a pathologist, parameter correction unit 31e adjusts the value specified for the specific parameter, such as the cell nucleus size, the staining density of a cell nucleus, the edge strength of the contour of a cell nucleus, or the feature like the likelihood ratio, to a value between the value given from the selected possible or possibles for a cell nucleus area and the value given from the unselected possible or possibles for a cell nucleus area (an arbitrary value between those values). Alternatively, parameter correction unit 31e adjusts the pattern to be determined as a dead cell, specified for the specific parameter, to the pattern extracted from the selected possible or possibles for a cell nucleus area. When receiving a result of the information-adding operation performed on the possible or possibles for a cell nucleus area by a pathologist, parameter correction unit 31 e adjusts the edge strength of the contour of a cell nucleus, specified for the specific parameter, to the difference in density between cell nuclei separated with a separation line.

The parameter setting unit 31a, pathological-diagnosis-image analysis unit 31b, display control unit 31c, operation control unit 31d and parameter correction unit 31e may be constituted as hardware devices. Alternatively, the parameter setting unit 31a, pathological-diagnosis-image analysis unit 31b, display control unit 31c, operation control unit 31d and parameter correction unit 31e may be provided as a software program (an image analysis program) stored in a computer-readable recording medium, which causes controller 31 to function as these components when being executed, and CPU32 may be configured to execute the image analysis program.

Storage unit 35 includes a HDD (Hard Disk Drive), SSD (Solid State Drive) or the like, and stores programs which cause CPU 32 to control the components of the device; information about processing and functions of the device; image data of a pathological diagnosis image; the basic parameter; the specific parameter; and others.

Network I/F unit 36 includes a NIC (Network Interface Card), a modem, or the like, so as to handle communication between the device and image acquisition device for pathological diagnosis 20 connected through communication network 40 and receive image data of a pathological diagnosis image and others from image acquisition device for pathological diagnosis 20.

Display unit 37 incudes a hardware device like a LCD (Liquid Crystal Display) or an organic EL (ElectroLuminescence) display, so as to display, according to instructions given by the above-described display control unit 31c, screens including a screen that shows possibles for a cell nucleus area of each group so that a selection from the possibles can be made, a screen that shows one or more possibles for a cell nucleus area so that an addition of information onto the one or more possibles can be made, a screen that shows one or more extracted cell nucleus as a final result, and a screen that allows an operator to perform setting or selection operations for the basic parameter or the specific parameter.

Operation unit 38 includes an input device like a keyboard, a mouse, a touch sensor composed of transparent electrodes arranged in a lattice, formed on display unit 37, or the like, so as to allow an operator to perform selection or information-adding operations on the possible or possibles for a cell nucleus area, and setting or selection operations for the basic parameter or the specific parameter.

FIG. 2 and FIGs. 3A and 3B illustrate an example of image processing system for pathological diagnosis 10 and image analysis device for pathological diagnosis 30, and the constitution and the control operations of each device can be modified appropriately. For example, image analysis device for pathological diagnosis 30 illustrated in FIGs. 3A and 3B is configured to make an extraction of possibles for a cell nucleus area by using the basic parameter, and a second extraction of one or more cell nucleus areas by using the specific parameter, but alternatively, a part of the functions of image analysis device for pathological diagnosis 30 may be transferred to another device communicatively connected to image analysis device for pathological diagnosis 30 so that another device can perform a part of the above-described operations. For example, a part of the functions of image analysis device for pathological diagnosis 30 may be transferred to another device (a server located in cloud computing environment) so that another device can make the extraction of possibles for a cell nucleus area by using the basic parameter and image analysis device for pathological diagnosis 30 can make just the second extraction of one or more cell nucleus areas by using the specific parameter.

Hereinafter, a description is given of concrete operations of image analysis device for pathological diagnosis 30. CPU 32 loads the image analysis program stored in ROM 33 or storage unit 35 onto RAM 34, and executes the program, thereby performing the processing of the steps illustrated in the flowchart of FIG. 4. The following description is given under the assumption that controller 31 (parameter setting unit 31a) has already set the basic parameter and the specific parameter and then recorded the parameters into storage unit 35 or another storage device.

First, controller 31 (pathological-diagnosis-image analysis unit 31b) of image analysis device for pathological diagnosis 30 acquires a pathological diagnosis image given by shooting a tissue specimen, from image acquisition device for pathological diagnosis 20 (S101). Controller 31 (pathological-diagnosis-image analysis unit 31b) then detects contours from the pathological diagnosis image by using a known method, and uses the detected contours to extract multiple possibles for a cell nucleus area from the pathological diagnosis image by using the basic parameter set in advance (S102).

Next, controller 31 (display control unit 31c) causes display unit 37 to display the multiple extracted possibles for a cell nucleus area so as to allow selection operations or information-adding operations thereon (S103). Controller 31 (operation control unit 31c) receives operations for correcting the specific parameter to a value corresponding to a pathologist, being operations to make a selection from the possibles or operations to make an addition of information onto the possibles, by a pathologist through operation unit 38 (S104). As described above, examples of the specific parameter include values of the cell nucleus size, the staining density of a cell nucleus, the edge strength of the contour of a cell nucleus, and the feature like the likelihood ratio; and the pattern to be determined as a dead cell.

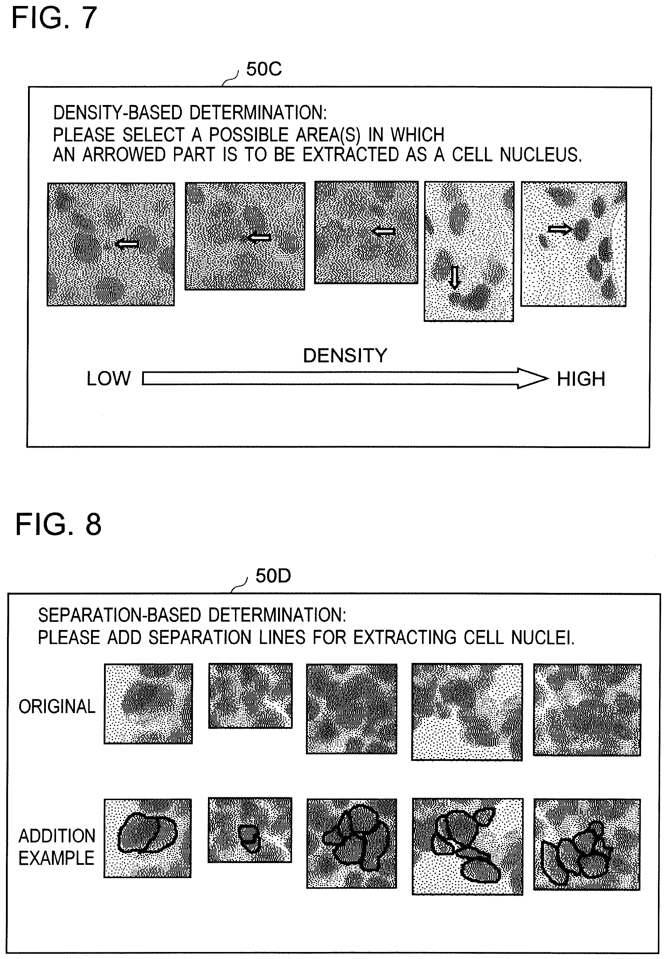

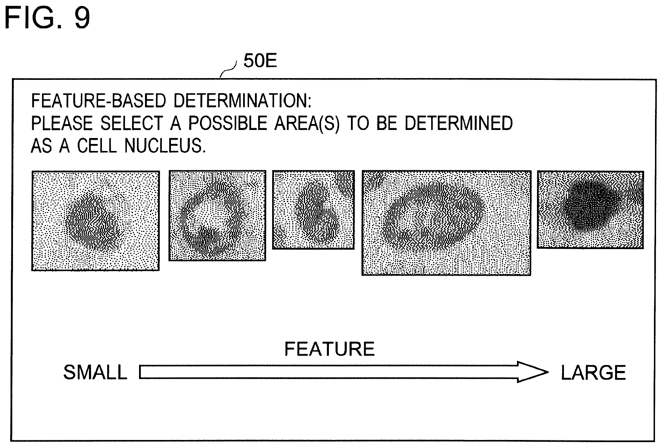

For example, controller 31 (display control unit 31c) causes display unit 37 to display a possible-area selection screen 50, thereby causing display unit 37 to display the extracted possibles for a cell nucleus area so that selection operations or information-adding operations can be performed on the extracted possibles. Controller 31 (operation control unit 31d) then receives the selection operations or information-adding operations performed on the possibles for a cell nucleus area by a pathologist though operation unit 38. For example, as shown in possible-area selection screen 50A illustrated in FIG. 5, controller 31 (display control unit 31c) determines possibles for a cell nucleus area each showing an object that can be determined as a dead cell, among the extracted possibles for a cell nucleus area; causes display unit 37 to display the determined possibles so that selection operations can be performed thereon; and prompts a pathologist to select one or more possibles each showing a cell nucleus area that the pathologist determined as a dead cell, through operation unit 38. In this process, the determination of the possibles for a cell nucleus area each showing an object that can be determined as a dead cell, may be made by, for example, fixing in advance a certain criterion or criteria by using at least one selected from values of the cell nucleus size, the shape of a cell nucleus, the staining density of a cell nucleus, the edge strength of the contour of a cell nucleus, and the feature like the likelihood ratio, and the pattern of a structure to be determined as a dead cell; and using the certain criterion or criteria to extract possibles for a cell nucleus area, each of which there is a high probability that the area indicates a dead cell, among the extracted possibles for a cell nucleus area. A pathologist selects two possibles for a cell nucleus area at the left and the center in possible-area selection screen 50A, in the case that the pathologist determines cells including the cell nuclei indicated in the possibles at the left and the center in possible-area selection screen 50A as dead cells because the cell nucleus has begun to rupture, and further determines a cell including the cell nucleus indicated in the possible for a cell nucleus area at the right in possible-area selection screen 50A as a non-dead cell because the cell nucleus is subject to vacuolar degeneration rather than rupture. For another example, as shown in possible-area selection screen 50B illustrated in FIG. 6, controller 31 (display control unit 31c) causes display unit 37 to display possibles for a cell nucleus area showing cell nuclei of various sizes among the extracted possibles so that selection operations can be performed thereon, and prompts a pathologist to select one or more possibles each showing a cell nucleus of the size to be extracted as a cell nucleus, through operation unit 38. For another example, as shown in possible-area selection screen 50C illustrated in FIG. 7, controller 31 (display control unit 31c) causes display unit 37 to display possibles for a cell nucleus area showing cell nuclei of various staining densities among the extracted possibles so that selection operations can be performed thereon, and prompts a pathologist to select one or more possibles each showing a cell nucleus of the staining densities to be extracted as a cell nucleus, through operation unit 38. For another example, similarly to the examples of FIGs. 5 to 7, controller 31 (display control unit 31c) causes display unit 37 to display possibles for a cell nucleus area showing cell nuclei having contours of various edge strengths among the extracted possibles so that selection operations can be performed thereon, and prompts a pathologist to select one or more possibles each showing a cell nucleus having the contour of the edge strength to be extracted as a cell nucleus, through operation unit 38. Alternatively, as shown in possible-area selection screen 50D illustrated in FIG. 8, controller 31 (display control unit 31c) causes display unit 37 to display one or more possibles for a cell nucleus area each indicating superimposed cell nuclei (see the original images in FIG. 8) among the extracted possibles so that adding-information operations can be performed thereon, and prompts a pathologist to add separation lines to separate superimposed cell nucleus onto each of the one or more displayed possibles through operation unit 38, with reference to addition examples. For another example, as shown in possible-area selection screen 50E illustrated in FIG. 9, controller 31 (display control unit 31c) causes display unit 37 to display possibles for a cell nucleus area showing cell nuclei having various values of the feature like the likelihood ratio among the extracted possibles so that selection operations can be performed thereon, and prompts a pathologist to select one or more possibles each showing an object to be extracted as a cell nucleus, through operation unit 38.

Returning to FIG. 4, controller 31 (parameter correction unit 31e) reads out a specific parameter set in advance, from storage unit 35 or another storage device; uses the result of the selection or the addition of information made on one or more possibles for a cell nucleus area by a pathologist, to correct the specific parameter to a value corresponding to the pathologist; and records the corrected specific parameter into storage unit 35 or another storage device (S105). For example, controller 31 (parameter correction unit 31e) analyzes a possible or possibles for a cell nucleus area selected on possible-area selection screen 50A illustrated in FIG. 5, to extract a pattern to be determined as a dead cell, and corrects the specific parameter by assigning the extracted pattern to the specific parameter. In concrete terms, controller 31 (parameter correction unit 31e) sets a pattern extracted from the selected possible or possibles (for example, a pattern for the contour of a structure indicating characteristics of a dead cell) to the specific parameter. In this process, controller 31 (parameter correction unit 31e) may add the pattern extracted from the selected possible or possibles to the specific parameter, or replace an existing pattern currently specified for the specific parameter with the pattern extracted from the selected possible or possibles. As another example, controller 31 (parameter correction unit 31e) compares a selected possible or possibles for a cell nucleus area and a unselected possible or possibles in possible-area selection screen 50B illustrated in FIG. 6, and corrects the specific parameter by assigning the value of the cell nucleus size between the cell nucleus size given from the selected possible or possibles and that given from the unselected possible or possibles, to the specific parameter. In concrete terms, controller 31 (parameter correction unit 31e) sets the value between the cell nucleus size given from the selected possible or possibles and that given from the unselected possible or possibles, to the specific parameter. As another example, controller 31 (parameter correction unit 31e) compares a selected possible or possibles for a cell nucleus area and an unselected possible or possibles in possible-area selection screen 50C illustrated in FIG. 7, and corrects the specific parameter by assigning the value of the staining density of a cell nucleus between the staining density of a cell nucleus given from the selected possible or possibles and that given from the unselected possible or possibles, to the specific parameter. In concrete terms, controller 31 (parameter correction unit 31e) sets the value between the staining density of a cell nucleus given from the selected possible or possibles and that given from the unselected possible or possibles, to the specific parameter. As another example, controller 31 (parameter correction unit 31e) uses the difference in density between cell nuclei separated with a separation line added on possible-area selection screen 50D illustrated in FIG. 8, to determine the edge strength of the contours of cell nuclei, and corrects the specific parameter by assigning the edge strength to the specific parameter. In concrete terms, controller 31 (parameter correction unit 31e) sets the value of the difference in density between cell nuclei separated with the separation line, to the specific parameter. Alternatively, in a case that possible-area selection screen 50 shows multiple sample images showing cell nuclei having contours of different edge strengths so that selection operations can be performed thereon, controller 31 (parameter correction unit 31e) may compare a selected possible or possibles for a cell nucleus area and an unselected possible or possibles in possible-area selection screen 50, and set the value between the an edge strength of a contour of a cell nucleus given from the selected possible or possibles and that given from the unselected possible or possibles, to the specific parameter. As another example, controller 31 (parameter correction unit 31e) compares a selected possible or possibles for a cell nucleus area and an unselected possible or possibles in possible-area selection screen 50E illustrated in FIG. 9, and corrects the specific parameter by assigning the value of the feature between the feature given from the selected possible or possibles and that given from the unselected possible or possibles, to the specific parameter. In concrete terms, controller 31 (parameter correction unit 31e) sets the value between the feature calculated from a cell nucleus or nuclei given from the selected possible or possibles and that given from the unselected possible or possibles, to the specific parameter.

Next, controller 31 (pathological-diagnosis-image analysis unit 31b) uses the corrected specific parameter to make a second extraction of one or more cell nucleus areas according to the criterion or criteria for cell nucleus determination made by the pathologist (S106). Controller 31 (display control unit 31c) then causes display unit 37 to display the pathological diagnosis image with the one or more extracted cell nucleus areas made recognizable, as a final result (S107), and ends a series of the processes of the pathological-diagnosis-image analysis.

As described above, controller 31 of image analysis device for pathological diagnosis 30 uses the basic parameter to extract multiple possibles for a cell nucleus area from the pathological diagnosis image; and causes display unit 37 to display one or more of the extracted possibles for a cell nucleus area so that operations to make a selection from the one or more possibles or operation to add information on to the one or more possibles can be made. Controller 31 further corrects, on the basis of a result of the selection operations or information-adding operations performed by a pathologist, the specific parameter to a value corresponding to the pathologist; records the corrected specific parameter into storage unit 35 or another storage device; and uses the corrected specific parameter to further extract one or more cell nucleus areas desired by the pathologist, from the possibles for a cell nucleus area. These operations allow appropriate extraction of a cell nucleus area or areas corresponding to a pathologist, from a pathological diagnosis image, with reducing the burden on pathologists.

### Second Embodiment

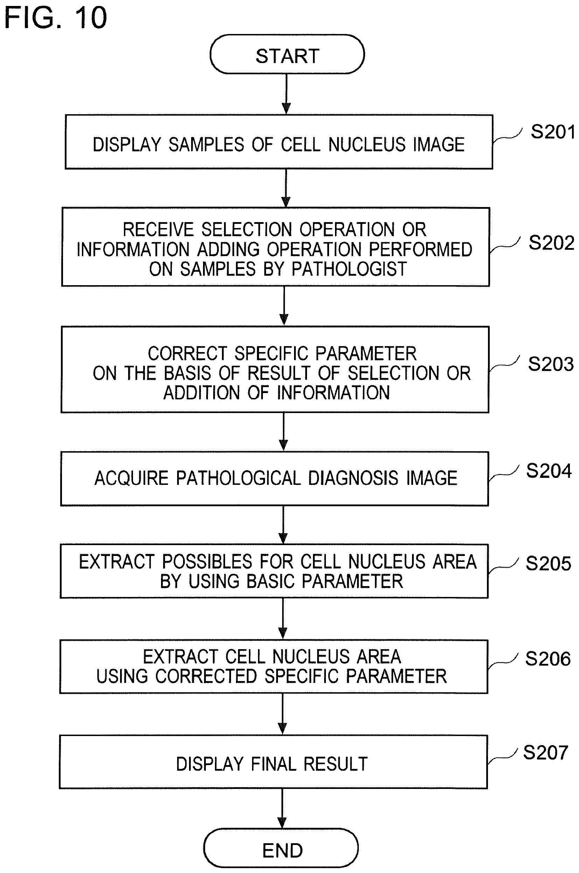

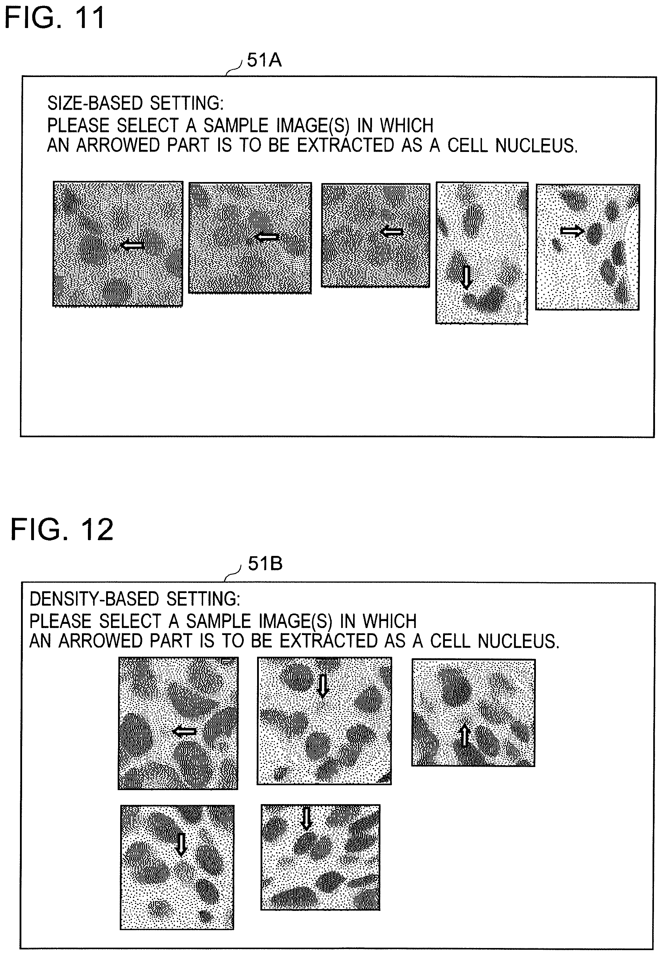

Next, a description is given of an image analysis program and an image analysis method according to a second embodiment of the present invention, with reference to FIG. 10 through FIG. 13. FIG. 10 is a flowchart illustrating operations of the image analysis device for pathological diagnosis. Each of FIGs. 11 to 13 is a diagram illustrating an example of a sample-image display screen for adjusting the specific parameter.

In the above-described first embodiment, one or more possibles for a cell nucleus area that were extracted using the basic parameter are displayed on display unit 37 so that selection operations or information-adding operations can be performed thereon, and the specific parameter is corrected on the basis of a result of the selection or the information addition made by a pathologist on the displayed possible or possibles for a cell nucleus area. Alternatively, before the extraction of one or more possibles for a cell nucleus area, the following operations may be performed: operations to present a question prepared in advance for adjusting the specific parameter (samples of a cell nucleus image) to a pathologist, so as to prompt the pathologist to answer; and to correct the specific parameter to a value corresponding to the pathologist on the basis of the answer.

The operations can be achieved by the image analysis device for pathological diagnosis 30 being the same in the basic constitution as that of first embodiment illustrated in FIG. 3A and 3B, but display control unit 31c, operation control unit 31d and parameter correction unit 31e perform the following operations.

Display control unit 31c classifies the samples of a cell nucleus image, that were prepared in advance for adjusting a determination criteria for each pathologist, into groups for adjusting the specific parameter to a value corresponding to a diagnostician like a pathologist. In this process, two or more groups may include the same sample of a cell nucleus image. Then, display control unit 31c causes display unit 37 to display the samples of a cell nucleus image in each group so that a selection from the samples can be made. Alternatively, display control unit 31c determines one or more samples of a cell nucleus image to be used for adjusting the specific parameter to a value corresponding to a diagnostician like a pathologist, and causes display unit 37 to display the one or more determined samples of a cell nucleus image so that an addition of information onto the one or more of the determined samples of a cell nucleus image can be made. Further, display control unit 31c, in response to extraction of one or more cell nucleus areas corresponding to a pathologist from possibles for a cell nucleus area with pathological-diagnosis-image analysis unit 31b, causes display unit 37 to display the pathological diagnosis image with the one or more extracted cell nucleus areas made recognizable, as a final result. Display control unit 31c further cause display unit 37 to display a screen for causing an operator to perform setting or selection operation for the basic parameter or the specific parameter, as needed. As the samples of a cell nucleus image, images selected from possible areas that were extracted in advance, or drawings each schematically representing a cell nucleus may be employed.

Operation control unit 31d, in response to selection operation performed on the samples of a cell nucleus image displayed by display unit 37, by a pathologist through operation unit 38, receives the selection operation and informs about the result of the selection operation to parameter correction unit 31e. Further, operation control unit 31d, in response to information-adding operation performed on the samples of a cell nucleus image displayed by display unit 37, by a pathologist through operation unit 38, receives the information-adding operation and informs about the result of the information-adding operation to parameter correction unit 31e. Operation control unit 31d may further receive setting operations and selection operations for the basic parameter or the specific parameter, as needed.

Parameter correction unit 31e receives a result of the selection operation or the information-adding operation performed on the one or more samples of a cell nucleus image by a pathologist, from operation control unit 31d. Parameter correction unit 31e corrects the specific parameter to a value corresponding to a pathologist on the basis of the difference between the selected sample or samples of a cell nucleus image and the unselected sample or samples of a cell nucleus image, or corrects the specific parameter to a value corresponding to a pathologist on the basis of the contents of the added information. Parameter correction unit 31e then records the corrected specific parameter into storage unit 35 or another storage device. For example, when receiving a result of the selection operation performed on the samples of a cell nucleus image by a pathologist, parameter correction unit 31 e adjusts the value specified for the specific parameter, such as the cell nucleus size, the staining density of a cell nucleus, the edge strength of the contour of a cell nucleus, or the feature like the likelihood ratio, to a value between the value given from the selected sample or samples of a cell nucleus image and the value given from the unselected sample or samples of a cell nucleus image (an arbitrary value between those values). Alternatively, parameter correction unit 31e adjusts the pattern to be determined as a dead cell, specified for the specific parameter, to the pattern extracted from the selected sample or samples of a cell nucleus image. When receiving a result of the information-adding operation performed on the sample or samples of a cell nucleus image by a pathologist, parameter correction unit 31e adjusts the edge strength of the contour of a cell nucleus, specified for the specific parameter, to the difference in density between cell nuclei separated with a separation line.

Hereinafter, a description is given of concrete operations of image analysis device for pathological diagnosis 30. CPU 32 loads the image analysis program stored in ROM 33 or storage unit 35 onto RAM 34, and executes the program, thereby performing the processing of the steps illustrated in the flowchart of FIG. 10. The following description is given under the assumption that samples of a cell nucleus image to be used for adjusting the specific parameter to a value corresponding to a diagnostician, have been recorded in storage unit 35 or another storage device in advance.

First, controller 31 (display control unit 31c) of image analysis device for pathological diagnosis 30 causes display unit 37 to display one or more samples of a cell nucleus image recorded in storage unit 35 or another storage device in advance (S201). Controller 31 (operation control unit 31d) then receives operations for correcting the specific parameter to a value corresponding to a pathologist, being operations to make a selection from the samples or operations to make an addition of information onto the sample or samples, by a pathologist through operation unit 38 (S202). As described above, examples of the specific parameter include values of the cell nucleus size, the staining density of a cell nucleus, the edge strength of the contour of a cell nucleus, and the feature like the likelihood ratio; and the pattern to be determined as a dead cell.

For example, controller 31 (display control unit 31c) causes display unit 37 to display a sample-image display screen 51, thereby causing display unit 37 to display one or more samples of a cell nucleus image so that selection operations or information-adding operations can be performed thereon. Controller 31 (operation control unit 31d) then receives the selection operations or information-adding operations performed on the one or more samples of a cell nucleus image by a pathologist though operation unit 38. For example, as shown in sample-image display screen 51A illustrated in FIG. 11, controller 31 (display control unit 31c) causes display unit 37 to display samples of a cell nucleus image showing cell nuclei of various sizes so that selection operations can be performed thereon, and prompts a pathologist to select one or more samples each showing a cell nucleus of the size to be extracted as a cell nucleus, through operation unit 38. For another example, as shown in sample-image display screen 51B illustrated in FIG. 12, controller 31 (display control unit 31c) causes display unit 37 to display samples of a cell nucleus image showing cell nuclei of various staining densities so that selection operations can be performed thereon, and prompts a pathologist to select one or more samples each showing a cell nucleus of the staining densities to be extracted as a cell nucleus, through operation unit 38. For another example, as shown in sample-image display screen 51C illustrated in FIG. 13, controller 31 (display control unit 31c) causes display unit 37 to display one or more samples of a cell nucleus image each indicating superimposed cell nuclei (see questions in FIG. 13) so that adding-information operations can be performed thereon, and prompts a pathologist to add separation lines to separate superimposed cell nucleus onto each of the one or more displayed samples through operation unit 38, with reference to answer examples. In FIGs. 11 to 13, actual pathological diagnosis images are illustrated as examples of samples of a cell nucleus image, but alternatively, schematic drawings to be used for adjusting the value of the cell nucleus size, the staining density of a cell nucleus, the edge strength of the contour of a cell nucleus, or the feature like a likelihood ratio, or a pattern to be determined as a dead cell, to a value corresponding to a pathologist may be displayed by display unit 37. Further, in FIGs. 11 to 13, the following screens displayed by display unit 37 are illustrated as examples: sample-image display screens each for setting one of the values of the cell nucleus size, the staining density of a cell nucleus, and the edge strength of the contour of a cell nucleus, but alternatively, controller 31 (display control unit 31c) may similarly cause display unit 37 to display multiple samples of a cell nucleus image showing various values of the feature calculated from cell nuclei, multiple samples of a cell nucleus image showing cell nuclei having contours of various edge strengths, or multiple samples of a cell nucleus image each showing an object that can be determined as a dead cell, so that selection operations can be performed thereon, to prompt a pathologist to select one or more samples of a cell nucleus image each showing an object to be extracted as a cell nucleus, through operation unit 38.

Returning to FIG. 10, controller 31 (parameter correction unit 31e) reads out a specific parameter set in advance, from storage unit 35 or another storage device, uses the result of the selection or the addition of information made on multiple samples by a pathologist, to correct the specific parameter to a value corresponding to the pathologist; and records the corrected specific parameter into storage unit 35 or another storage device (S203). For example, controller 31 (parameter correction unit 31e) compares a selected sample or samples of a cell nucleus image and a unselected sample or samples of a cell nucleus image in sample-image display screen 51A illustrated in FIG. 11, and corrects the specific parameter by assigning the value of the cell nucleus size between the cell nucleus size given from the selected sample or samples and that given from the unselected sample or samples, to the specific parameter. In concrete terms, controller 31 (parameter correction unit 31e) sets the value between the cell nucleus size given from the selected sample or samples and that given from the unselected sample or samples, to the specific parameter. As another example, controller 31 (parameter correction unit 31e) compares a selected sample or samples and an unselected sample or samples in sample-image display screen 51B illustrated in FIG. 12, and corrects the specific parameter by assigning the value of the staining density of a cell nucleus between the staining density of a cell nucleus given from the selected sample or samples and that given from the unselected sample or samples, to the specific parameter. In concrete terms, controller 31 (parameter correction unit 31e) sets the value between the staining density of a cell nucleus given from the selected sample or samples and that given from the unselected sample or samples, to the specific parameter. As another example, controller 31 (parameter correction unit 31e) uses the difference in density between cell nuclei separated with a separation line added on sample-image display screen 51C illustrated in FIG. 13, to determine the edge strength of the contours of cell nuclei, and corrects the specific parameter by assigning the edge strength to the specific parameter. In concrete terms, controller 31 (parameter correction unit 31e) sets the value of the difference in density between cell nuclei separated with the separation line, to the specific parameter. Alternatively, in a case that sample-image display screen 51 shows multiple samples showing cell nuclei having contours of different edge strengths so that selection operations can be performed thereon, controller 31 (parameter correction unit 31e) may compare a selected sample or samples and an unselected sample or samples in sample-image display screen 51, and set the value between the an edge strength of a contour of a cell nucleus given from the selected sample or samples and that given from the unselected sample or samples, to the specific parameter. As another example, in a case that sample-image display screen 51 shows multiple samples showing various values of the feature so that selection operations can be performed thereon, controller 31 (parameter correction unit 31e) compares a selected sample or samples and an unselected sample or samples in sample-image display screen 51, and corrects the specific parameter by assigning the value of the feature between the feature given from the selected sample or samples and that given from the unselected sample or samples, to the specific parameter. In concrete terms, controller 31 (parameter correction unit 31e) sets the value between the feature calculated from a cell nucleus or nuclei given from the selected sample or samples and that given from the unselected sample or samples, to the specific parameter. As anther example, in a case that sample-image display screen 51 shows multiple samples of a cell nucleus image each showing an object that can be determined as a dead cell so that selection operations can be performed thereon, controller 31 (parameter correction unit 31e) may analyze the sample or samples selected on sample-image display screen 51, to extract a pattern to be determined as a dead cell, and corrects the specific parameter by assigning the extracted pattern to the specific parameter. In concrete terms, controller 31 (parameter correction unit 31e) sets a pattern extracted from the selected sample or samples of a cell nucleus image (for example, a pattern for the contour of a structure indicating characteristics of a dead cell) to the specific parameter. In this process, controller 31 (parameter correction unit 31e) may add the pattern extracted from the selected sample or samples of a cell nucleus image to the specific parameter, or replace an existing pattern currently specified for the specific parameter with the pattern extracted from the sample or samples of a cell nucleus image.

Next, controller 31 (pathological-diagnosis- image analysis unit 31b) acquires a pathological diagnosis image given by shooting a tissue specimen, from image acquisition device for pathological diagnosis 20 (S204). Controller 31 (pathological-diagnosis-image analysis unit 31b) then detects contours from the pathological diagnosis image by using a known method, and uses the detected contours to extract multiple possibles for a cell nucleus area from the pathological diagnosis image by using the basic parameter set in advance (S205).

Next, controller 31 (pathological-diagnosis- image analysis unit 31b) uses the corrected specific parameter to make a second extraction of one or more cell nucleus areas according to the criteria for cell nucleus determination made by the pathologist (S206). Controller 31 (display control unit 31c) then causes display unit 37 to display the pathological diagnosis image with the one or more extracted cell nucleus areas made recognizable, as a final result (S207), and ends a series of the processes of the pathological-diagnosis-image analysis.

As described above, controller 31 of pathological-diagnosis-image analysis device 30 causes display unit 37 to display samples of a cell nucleus image prepared in advance so that selection operations or information-adding operations can be performed thereon; corrects the specific parameter to a value corresponding to a pathologist on the basis of a result of the selection or addition of information made by the pathologist; and records the corrected specific parameter into storage unit 35 or another storage device. After that, controller 31 uses the basic parameter to extract multiple possibles for a cell nucleus area from pathological diagnosis image; and uses the corrected specific parameter to further extract one or more cell nucleus areas desired by the pathologist, from the possibles for a cell nucleus area. These operations allow appropriate extraction of a cell nucleus area or areas corresponding to a pathologist, from a pathological diagnosis image, with reducing the burden on pathologists.

The present invention is not limited to the above-described embodiments, without departing from the spirit and scope of the present invention, the constitution and control contents of the image analysis device for pathological diagnosis 30 can be changed appropriately.

For example, images for pathological diagnosis used in the above-described embodiments are examples for illustrative purpose only, and arbitrary images for the pathological diagnosis may be used.

For another example, in the first embodiment, after extracting possibles for a cell nucleus area, controller 31 of image analysis device for pathological diagnosis 30 corrects the specific parameter to a value corresponding to a pathologist; in the second embodiment, before extracting possibles for a cell nucleus area, controller 31 of image analysis device for pathological diagnosis 30 corrects the specific parameter to a value corresponding to a pathologist. Alternatively, controller 31 may correct the specific parameter at two stages before and after the extraction of possibles for a cell nucleus area.

Further, in the above-described embodiments, descriptions were given of the analysis of images for pathological diagnosis, but alternatively, the image analysis method according to the present invention can be similarly applied to image analyses in which a determination criterion varies depending on a user.

### Industrial Applicability

The present invention is applicable to image analysis programs for extracting a diagnosis target like a cell nucleus, corresponding to a diagnostician like a pathologist, from an image for pathological diagnosis, recording media each storing the image analysis program, and image analysis methods.

### Reference Signs List

10: image processing system for pathological diagnosis
20: image acquisition device for pathological diagnosis
30, AP: image analysis device for pathological diagnosis
31: controller
31a: parameter setting unit
31b: pathological-diagnosis-image analysis unit
31c: display control unit
31d: operation control unit
31e: parameter correction unit
32: CPU
33: ROM
34: RAM
35: storage unit
36: network I/F unit
37: display unit
38: operation unit
40: communication networks
50, 50A, 50B, 50C, 50D, 50E: possible-area display screen
51, 51A, 51B, and 51C: sample-image display screen

## Claims

1. An image analysis program to be executed in a device that includes a display unit, an operation unit and a storage unit, for analyzing images for pathological diagnosis by using a first parameter for determining a criterion for cell nucleus determination for each diagnosis purpose and a second parameter for determining a criterion for cell nucleus determination for each diagnostician, both recorded in the storage unit, the program comprising instructions which cause the device to carry out:
a step of first analyzing a pathological diagnosis image, including acquiring a pathological diagnosis image prepared by shooting a tissue specimen, and extracting a plurality of possibles for a cell nucleus area from the pathological diagnosis image, by using the first parameter;
a step of second analyzing a pathological diagnosis image, including further extracting one or more cell nucleus areas from the plurality of possibles for a cell nucleus area that were extracted, by using the second parameter; and
a step of first carrying out display control, including causing the display unit to display the pathological diagnosis image with the one or more cell nucleus areas that were extracted made recognizable.

2. The image analysis program of claim 1, further comprising instructions which cause the device to carry out:
a step of second carrying out display control, including causing the display unit to display one or more of the plurality of possibles for a cell nucleus area that were extracted, so that a selection from the one or more of the plurality of possibles or an addition of information onto the one or more of the plurality of possibles can be made,
a step of carrying out operations control, including receiving an operation to make a selection from the one or more of the plurality of possibles or an operation to make an addition of information onto the one or more of the plurality of possibles, by using the operation unit, and
a step of making a parameter correction, including using a result of the operation to make a selection from the one or more of the plurality of possibles or a result of the operation to make an addition of information onto the one or more of the plurality of possibles, to correct the second parameter, and recording the second parameter into the storage unit,
wherein the step of second carrying out display control, the step of carrying out operations control and the step of making a parameter correction are carried out after the step of first analyzing a pathological diagnosis image, and
in the step of second analyzing a pathological diagnosis image, one or more cell nucleus areas are extracted from the plurality of possibles for a cell nucleus area that were extracted, by using the corrected second parameter.

3. The image analysis program of claim 1, further comprising instructions which cause the device to carry out:
a step of second carrying out display control, including causing the display unit to display one or more samples of a cell nucleus image, so that a selection from the one or more samples of a cell nucleus image or an addition of information onto the one or more samples of a cell nucleus image can be made,
a step of carrying out operations control, including receiving an operation to make a selection from the one or more samples of a cell nucleus image or an operation to make an addition of information onto the one or more samples of a cell nucleus image, by using the operation unit, and
a step of making a parameter correction, including using a result of the operation to make a selection from the one or more samples of a cell nucleus image or a result of the operation to make an addition of information onto the one or more samples of a cell nucleus image, to correct the second parameter, and recording the second parameter into the storage unit,
wherein the step of second carrying out display control, the step of carrying out operations control and the step of making a parameter correction are carried out before the step of first analyzing a pathological diagnosis image, and
in the step of second analyzing a pathological diagnosis image, one or more cell nucleus areas are extracted from the plurality of possibles for a cell nucleus area that were extracted, by using the corrected second parameter.

4. The image analysis program of claim 2 or 3, wherein
the second parameter is a cell nucleus size,
in the step of second carrying out display control, the display unit is caused to display two or more of the plurality of possibles for a cell nucleus area or two or more samples of a cell nucleus image, so that a selection from the two or more of the plurality of possibles or the two or more samples can be made, where the displayed possibles or samples show cell nuclei of different cell nucleus sizes, and
in the step of making a parameter correction, a value between a cell nucleus size given from a selected possible or possibles or a selected sample or samples and a cell nucleus size given from a unselected possible or possibles or a unselected sample or samples, is set to the second parameter.

5. The image analysis program of claim 2 or 3, wherein
the second parameter is a staining density of a cell nucleus,
in the step of second carrying out display control, the display unit is caused to display two or more of the plurality of possibles for cell nucleus areas or two or more samples of a cell nucleus image, so that a selection from the two or more of the plurality of possibles or the two or more samples can be made, where the displayed possibles or samples show cell nuclei of different staining densities, and
in the step of making a parameter correction, a value between a staining density of a cell nucleus given from a selected possible or possibles or a selected sample or samples and a staining density of a cell nucleus given from a unselected possible or possibles or a unselected sample or samples, is set to the second parameter.

6. The image analysis program of claim 2 or 3, wherein
the second parameter is a feature calculated from a cell nucleus,
in the step of second carrying out display control, the display unit is caused to display two or more of the plurality of possibles for cell nucleus areas or two or more samples of a cell nucleus image, so that a selection from the two or more of the plurality of possibles or the two or more samples can be made, where the displayed possibles or samples show different features each calculated from a cell nucleus, and
in the step of making a parameter correction, a value between a feature calculated from a cell nucleus or nuclei given from a selected possible or possibles or a selected sample or samples and a feature calculated from a cell nucleus or nuclei given from a unselected possible or possibles or a unselected sample or samples, is set to the second parameter.

7. The image analysis program of claim 2 or 3, wherein
the second parameter is an edge strength of a contour of a cell nucleus,
in the step of second carrying out display control, the display unit is caused to display two or more of the plurality of possibles for cell nucleus areas or two or more samples of a cell nucleus image, so that a selection from the two or more of the plurality of possibles or the two or more samples can be made, where the displayed possibles or samples show cell nuclei having contours of different edge strengths, and
in the step of making a parameter correction, a value between an edge strength of a contour of a cell nucleus given from a selected possible or possibles or a selected sample or samples and an edge strength of a contour of a cell nucleus given from a unselected possible or possibles or a unselected sample or samples, is set to the second parameter.

8. The image analysis program of claim 2 or 3, wherein
the second parameter is an edge strength of a contour of a cell nucleus,
in the step of second carrying out display control, the display unit is caused to display one or more of the plurality of possibles for cell nucleus areas or one or more samples of a cell nucleus image, so that an addition of a separation line onto the one or more of the plurality of possibles or the one or more samples can be made, and
in the step of making a parameter correction, a value between densities of cell nuclei separated with the separation line, is set to the second parameter.

9. The image analysis program of claim 2 or 3, wherein
the second parameter is a pattern to be determined as a dead cell,
in the step of second carrying out display control, the display unit is caused to display two or more of the plurality of possibles for cell nucleus areas or two or more samples of a cell nucleus image, in which a dead cell can be determined, so that a selection from the two or more of the plurality of possibles or the two or more samples can be made, and
in the step of making a parameter correction, a pattern extracted from a selected possible or possibles or a selected sample or samples, is set to the second parameter.

10. An image analysis method for use in a device that includes a display unit, an operation unit and a storage unit, for analyzing images for pathological diagnosis by using a first parameter for determining a criterion for cell nucleus determination for each diagnosis purpose and a second parameter for determining a criterion for cell nucleus determination for each diagnostician, both recorded in the storage unit, the image analysis method comprising:
a step of first analyzing a pathological diagnosis image, including acquiring a pathological diagnosis image prepared by shooting a tissue specimen, and extracting a plurality of possibles for a cell nucleus area from the pathological diagnosis image, by using the first parameter;
a step of second analyzing a pathological diagnosis image, including further extracting one or more cell nucleus areas from the plurality of possibles for a cell nucleus area that were extracted, by using the second parameter; and
a step of first carrying out display control, including causing the display unit to display the pathological diagnosis image with the one or more cell nucleus areas that were extracted made recognizable.

11. The image analysis method of claim 10, further comprising:
a step of second carrying out display control, including causing the display unit to display one or more of the plurality of possibles for a cell nucleus area that were extracted, so that a selection from the one or more of the plurality of possibles or an addition of information onto the one or more of the plurality of possibles can be made,
a step of carrying out operations control, including receiving an operation to make a selection from the one or more of the plurality of possibles or an operation to make an addition of information onto the one or more of the plurality of possibles, by using the operation unit, and
a step of making a parameter correction, including using a result of the operation to make a selection from the one or more of the plurality of possibles or a result of the operation to make an addition of information onto the one or more of the plurality of possibles, to correct the second parameter, and recording the second parameter into the storage unit,
wherein the step of second carrying out display control, the step of carrying out operations control and the step of making a parameter correction are carried out after the step of first analyzing a pathological diagnosis image, and
in the step of second analyzing a pathological diagnosis image, one or more cell nucleus areas are extracted from the plurality of possibles for a cell nucleus area that were extracted, by using the corrected second parameter.

12. The image analysis method of claim 10, further comprising:
a step of second carrying out display control, including causing the display unit to display one or more samples of a cell nucleus image, so that a selection from the one or more samples of a cell nucleus image or an addition of information onto the one or more samples of a cell nucleus image can be made,
a step of carrying out operations control, including receiving an operation to make a selection from the one or more samples of a cell nucleus image or an operation to make an addition of information onto the one or more samples of a cell nucleus image, by using the operation unit, and
a step of making a parameter correction, including using a result of the operation to make a selection from the one or more samples of a cell nucleus image or a result of the operation to make an addition of information onto the one or more samples of a cell nucleus image, to correct the second parameter, and recording the second parameter into the storage unit,
wherein the step of second carrying out display control, the step of carrying out operations control and the step of making a parameter correction are carried out after the step of first analyzing a pathological diagnosis image, and
in the step of second analyzing a pathological diagnosis image, one or more cell nucleus areas are extracted from the plurality of possibles for a cell nucleus area that were extracted, by using the corrected second parameter.

13. The image analysis method of claim 11 or 12, wherein
the second parameter is a cell nucleus size,
in the step of second carrying out display control, the display unit is caused to display two or more of the plurality of possibles for a cell nucleus area or two or more samples of a cell nucleus image, so that a selection from the two or more of the plurality of possibles or the two or more samples can be made, where the displayed possibles or samples show cell nuclei of different cell nucleus sizes, and
in the step of making a parameter correction, a value between a cell nucleus size given from a selected possible or possibles or a selected sample or samples and a cell nucleus size given from a unselected possible or possibles or a unselected sample or samples, is set to the second parameter.

14. The image analysis method of claim 11 or 12, wherein
the second parameter is a staining density of a cell nucleus,
in the step of second carrying out display control, the display unit is caused to display two or more of the plurality of possibles for cell nucleus areas or two or more samples of a cell nucleus image, so that a selection from the two or more of the plurality of possibles or the two or more samples can be made, where the displayed possibles or samples show cell nuclei of different staining densities, and
in the step of making a parameter correction, a value between a staining density of a cell nucleus given from a selected possible or possibles or a selected sample or samples and a staining density of a cell nucleus given from a unselected possible or possibles or a unselected sample or samples, is set to the second parameter.

15. The image analysis method of claim 11 or 12, wherein
the second parameter is a feature calculated from a cell nucleus,
in the step of second carrying out display control, the display unit is caused to display two or more of the plurality of possibles for cell nucleus areas or two or more samples of a cell nucleus image, so that a selection from the two or more of the plurality of possibles or the two or more samples can be made, where the displayed possibles or samples show different features each calculated from a cell nucleus, and
in the step of making a parameter correction, a value between a feature calculated from a cell nucleus or nuclei given from a selected possible or possibles or a selected sample or samples and a feature calculated from a cell nucleus or nuclei given from a unselected possible or possibles or a unselected sample or samples, is set to the second parameter.

16. The image analysis method of claim 11 or 12, wherein
the second parameter is an edge strength of a contour of a cell nucleus,
in the step of second carrying out display control, the display unit is caused to display two or more of the plurality of possibles for cell nucleus areas or two or more samples of a cell nucleus image, so that a selection from the two or more of the plurality of possibles or the two or more samples can be made, where the displayed possibles or samples show cell nuclei having contours of different edge strengths, and
in the step of making a parameter correction, a value between an edge strength of a contour of a cell nucleus given from a selected possible or possibles or a selected sample or samples and an edge strength of a contour of a cell nucleus given from a unselected possible or possibles or a unselected sample or samples, is set to the second parameter.

17. The image analysis method of claim 11 or 12, wherein
the second parameter is an edge strength of a contour of a cell nucleus,
in the step of second carrying out display control, the display unit is caused to display one or more of the plurality of possibles for cell nucleus areas or one or more samples of a cell nucleus image, so that an addition of a separation line onto the one or more of the plurality of possibles or the one or more samples can be made, and
in the step of making a parameter correction, a value between densities of cell nuclei separated with the separation line, is set to the second parameter.

18. The image analysis method of claim 11 or 12, wherein
the second parameter is a pattern to be determined as a dead cell,
in the step of second carrying out display control, the display unit is caused to display two or more of the plurality of possibles for cell nucleus areas or two or more samples of a cell nucleus image, in which a dead cell can be determined, so that a selection from the two or more of the plurality of possibles or the two or more samples can be made, and
in the step of making a parameter correction, a pattern extracted from a selected possible or possibles or a selected sample or samples, is set to the second parameter.
